# EUROPEAN PATENT SPECIFICATION

(11) **EP 3 936 174 B1**
(45) Date of publication and mention of the grant of the patent: **15.10.2025**
(21) Application number: 20305769.0
(22) Date of filing: 07.07.2020
(51) Int. Cl.: A61M 5/34, A61M 5/48

(54) **DEVICE FOR FLUIDLY CONNECTING A MEDICAL CONTAINER TO A CONNECTOR AND METHOD FOR MANUFACTURING SAID DEVICE**
VORRICHTUNG ZUM FLUIDISCHEN VERBINDEN EINES MEDIZINISCHEN BEHÄLTERS MIT EINEM VERBINDER UND VERFAHREN ZUR HERSTELLUNG DER VORRICHTUNG
DISPOSITIF PERMETTANT DE RELIER DE MANIÈRE FLUIDE UN RÉCIPIENT MÉDICAL À UN CONNECTEUR ET PROCÉDÉ DE FABRICATION DUDIT DISPOSITIF

(43) Date of publication of application: 12.01.2022
(73) Proprietor: Becton Dickinson France, 38800 Le Pont-de-Claix (FR); Becton Dickinson Holdings Pte. Ltd., Singapore 639461 (SG)
(72) Inventor: Deeliprao Jadhav, Amarsinh, 50005 Karnataka (IN)
(74) Representative: Germain Maureau

(56) References cited:
- EP-A2- 0 934 757
- US-A1- 2010 217 209
- US-A1- 2014 194 851

## Description

The invention relates to a device configured to be plugged to a medical container, such as a syringe, and to a connector, such as a vascular access device, a catheter, or a needle hub, for infusion or injection of a medical fluid to a patient. The invention also relates to a medical container and a connector comprising said device and to method for manufacturing said device.

In this application, the distal end of a component or of a device is to be understood as meaning the end closest to the patient and the proximal end is to be understood as meaning the end furthest to the patient. Likewise, in this application, the "distal direction" is to be understood as meaning the direction of infusion or injection, and the "proximal direction" is to be understood as meaning the opposite direction to said direction of infusion or injection, that is to say the direction away from the patient.

In general, medical injection device are used to inject through a body a limited amount of a pharmaceutical composition. When larger amounts or volumes of a pharmaceutical composition are to be infused in a body, one may use vascular access devices. Such devices are inserted into veins via peripheral or central vessels. Vascular access devices can be used for infusing fluid (e.g., saline solution, blood, medicaments, and/or total parenteral nutrition) into a patient body, withdrawing fluids (e.g., blood) from a patient body, and/or monitoring various parameters of the patient's vascular system.

However, vascular access devices can become occluded. To ensure vascular access devices are used properly and do not become occluded, standards of practice have been developed. These standards include a cleaning procedure, which is commonly referred to as a flush procedure. These flush procedures maintain the patency of the vascular access device.

Flush procedures may be enhanced by use of a syringe specifically designed to generate lower injection pressure, such as for instance a 10 mL-volume syringe barrel, or by use of a "push-pause" or pulsatile flushing technique to remove debris or residue in the catheter that may cause occlusion or other undesirable effects. However these procedures are difficult and fastidious to follow for the healthcare practitioner, and may lead to too fast injection of the pharmaceutical composition into a patient's vein.

Fast injection of said pharmaceutical composition into peripheral intravenous lines may lead to temporary pressure build-up within the catheter and within the vein where the catheter is sited. This too high pressure may lead to vein damage such as rupture or collapsing. Moreover, the medical composition to be infused may infiltrate or leak outside the infusion device, causing clinical complications and the need to replace the infusion device such as the peripheral intravenous catheter, and/or change the infusion site.

There is therefore a need for a drug delivery device enabling ensuring a flush injection pressure that stays below a predetermined threshold in order to avoid the above-mentioned drawbacks.

The document US2003078534 proposes to minimize subjective pain response and any potential tissue damage to a patient resulting from of inappropriate pressures produced during the administration of a drug via hypodermic needle. However, the device is power-driven and includes multiple components such as a drive mechanism and a sensor to determine the internal pressure generated during the injection process, thereby leading to more complex use, high costs and a cumbersome device.

The document US20140194851 further discloses a flow actuated valve for implantable drug delivery device.

In this context, an object of the present invention is to provide a device that alleviates the above-mentioned drawbacks by limiting the infusion pressure from a syringe to a vascular access device, thereby avoiding damage to blood vessels, while being compact and easy to handle and manufacture.

As defined in claim 1, the invention is a device, such as a medical device, for fluidly connecting a medical container containing a medical fluid to a connector, wherein the device includes
a housing, said housing defining an internal conduit for circulation of the medical fluid from a fluid inlet to a fluid outlet,
a slider arranged inside the internal conduit such that the slider is movable along a central longitudinal axis A between an opening position, wherein the medical fluid is allowed to flow through the fluid outlet, and a closing position, wherein the medical fluid is prevented from flowing through said fluid outlet,
a plug connected to the slider, said plug being configured to close the fluid outlet when the slider is in the closing position,
a resilient member disposed proximally to the plug and configured to exert a proximal force on said the slider such that the resilient member maintains the slider in the opening position, and
a proximally oriented pressure area provided on an actuation member of the slider, said pressure area being configured to cause the slider to reach the closing position against the action of the resilient member when the medical fluid pressure exerted upon said pressure area is equal to or higher than a predetermined threshold, thereby causing the plug to close the fluid outlet.

The medical device of the invention therefore allows limiting the infusion pressure in the vascular access device. This prevents damage to the blood vessels. The pressure limiting mechanism of device is not motor driven and requires a limited number of components, thereby improving compactness and limiting costs.

In an embodiment, the actuation member includes a peripheral edge configured to guide the sliding movement of the slider between the opening and the closing positions

In an embodiment, the pressure area delimits at least two through-holes allowing the medical fluid to pass through the actuation member.

Preferably, the at least two through-holes are symmetric relative to a longitudinal plane containing the central longitudinal axis A.

This provides a symmetric flow path through pressure area and thus a uniform pressure onto the pressure area in order to avoid any tilting movement of the slider or any blockage, thus improving reliability of the device.

Preferably, the actuation member is disk shaped.

Preferably, the pressure area is orthogonal to the central longitudinal axis A.

As defined in claim 1, the slider includes a connecting rod extending along the central longitudinal axis A, said connecting rod connecting the actuation member to the plug.

Preferably, the actuation member is integral with the connecting rod such that the actuation member and the connecting rod form a single piece.

Preferably, the resilient member is a bellow extending around the connecting rod. As defined in claim 1, the device includes a holder, said holder including a guiding element for guiding the connecting rod along the central longitudinal axis A, an outer ring forming part of the housing, and holding elements connecting the guiding element to the outer ring such that the holding elements delimit openings for allowing the medical fluid to flow through the holder.

Preferably, the resilient member extends between the actuation member and the guiding element of the holder.

Preferably, the housing comprises a top housing and a bottom housing, the outer ring of the holder forming an intermediate housing portion interposed between the top and the bottom housings.

In an embodiment, the plug is made of a softer material than the slider.

Another aspect of the invention is a medical container comprising a reservoir for containing a medical fluid and the above-described device fluidly connected to the reservoir of said medical container.

Another aspect of the invention is a method for manufacturing the above-described device, said method comprising the steps of:
- assembling the holder, slider, the resilient member and the plug;
- securing the holder to the bottom housing, preferably by ultrasonic welding;
- securing the holder to the top housing, preferably by ultrasonic welding.

The invention and the advantages arising therefrom will clearly emerge from the detailed description that is given below with reference to the appended drawings as follows:
- Figure 1 is a perspective view of a device according an embodiment of the invention,
- Figure 2 is a cross-section view in a longitudinal plane of a device according an embodiment of the invention,
- Figure 3 is an exploded view of a device according an embodiment of the invention,
- Figure 4 is a cross-section view in a longitudinal plane illustrating an operation of a device according to an embodiment of the invention,
- Figure 5 is a semi-transparent perspective view of a device according an embodiment of the invention,
- Figure 6 is a cross-section view in a longitudinal plane illustrating an operation of a device according to an embodiment of the invention,
- Figure 7 is a perspective view of a medical container and a portion of the vascular access device according to an embodiment of the invention.

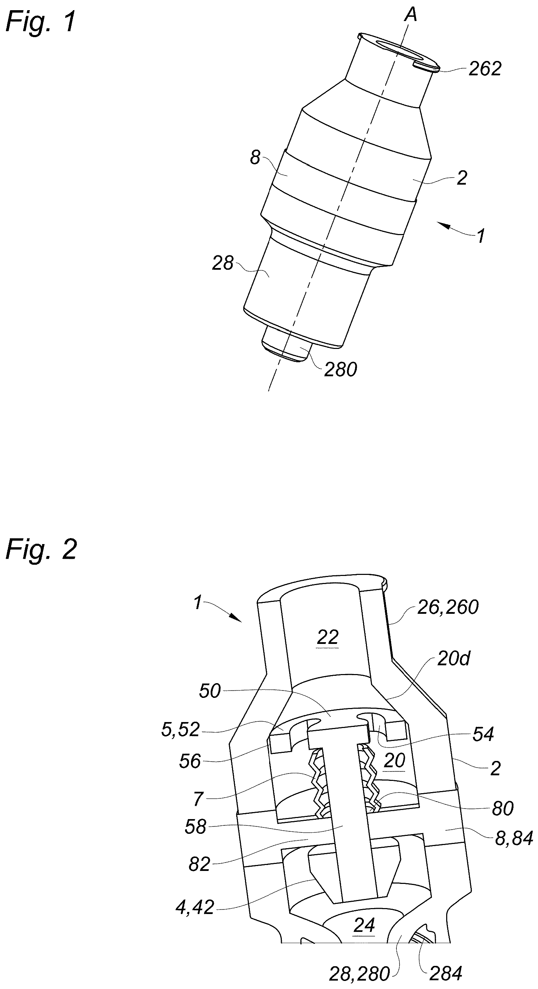

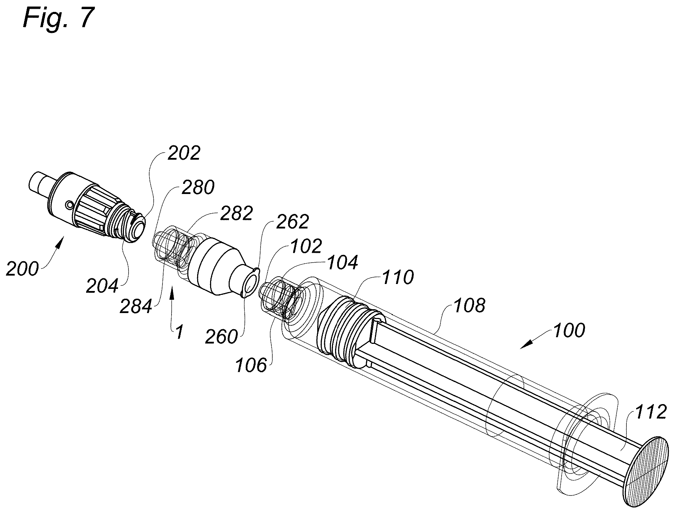

With reference to Figure 1 is shown a device 1 according to an embodiment of the invention. As illustrated on Figure 7, the device 1 is configured to be connected to a medical container 100, such as prefilled or pre-fillable syringe, and to a connector 200, such as a needle hub or a vascular access device, for instance an IV (intravenous) catheter, such that the medical fluid contained in the medical container 100 passes through the device 1 before reaching the connector 200. It is contemplated that the device 1 is an add-on device. Therefore, the medical container 100 may be connected to the connector 200 without the device 1 of the invention. The medical container 100 typically includes a barrel 108 defining a reservoir containing a medical fluid and a distal tip 102 defining a passageway in fluid communication with said reservoir. The medical container 100 may comprise a stopper 110 and a plunger rod 112 for expelling the medical product through the distal tip 102.

With reference to Figures 1 and 2, the add-on device 1 may include a housing 2 defining an internal conduit 20 for circulation of the medical product through the device 1 from a fluid inlet 22 to a fluid outlet 24. The housing 2 has a proximal end 26 provided with proximal connecting means, such as a thread or wings 262, for connecting the device 1 to the medical container 100 such that the medical fluid contained in the medical container 100 flows from the medical container 100 to the device 1 via the fluid inlet 22, and a distal end 28 provided with distal connecting means, such as a thread 284, for connecting the device 1 to a connector 200 such that the medical fluid flows from the device 1 to said connector 200 via the fluid outlet 24.

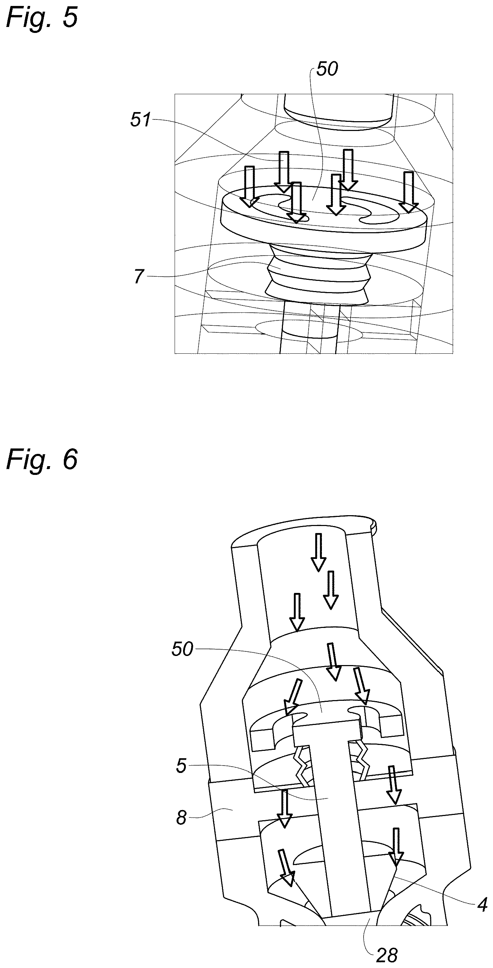

The device 1 further includes a plug 4 for closing the fluid outlet 24, a slider 5 that is movable together with the plug 4 between an opening position, wherein the plug 4 is remote from the fluid outlet 24 (Figure 2) and a closing position (Figure 6), wherein the plug 4 closes the fluid outlet 24, a resilient member 7 urging the slider 5 and the plug 4 towards the opening position, and a holder 8 for guiding a sliding movement of the slider 5 between the opening and the closing positions along a central longitudinal axis A of the device 1.

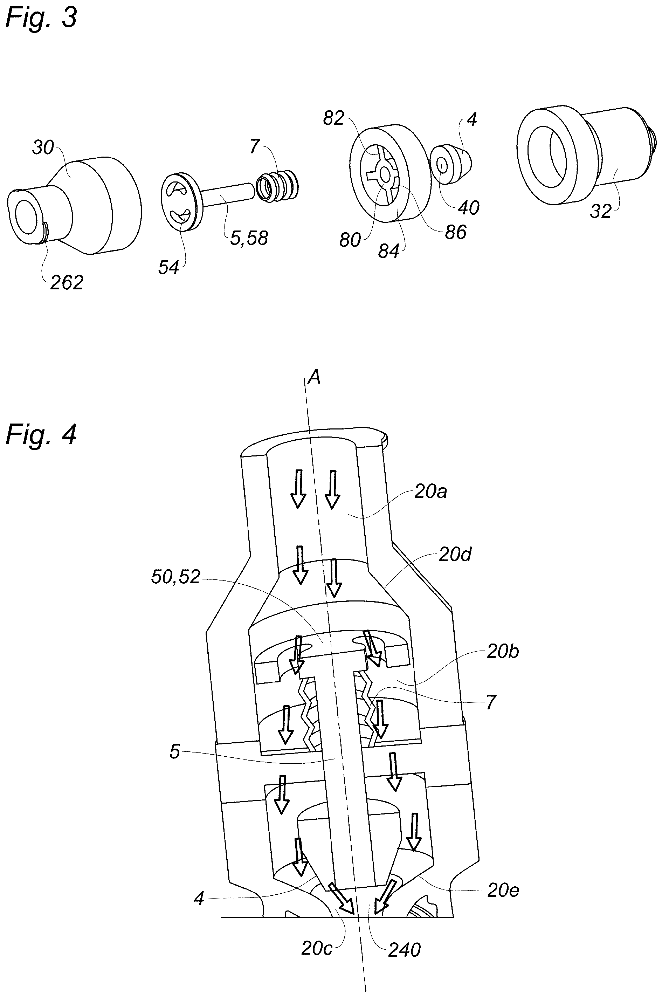

With reference to Figures 4 to 6, the slider 5 comprises a pressure area 50 arranged in the proximal part of the flow path of the medical fluid such that the medical fluid circulating inside the internal conduit 20 exerts a distal pressure onto the pressure area 50. The pressure area 50 is configured so that the medical fluid pressure exerted thereon causes the slider 5 and the plug 4 to move distally against the action of the resilient member 7 towards the closing position.

As long as the pressure remains below a predetermined threshold, the resilient member 7 maintains the plug 4 away from the fluid outlet 24 and the slider 5 remains in an intermediate position (Figure 4) and does not reach the closing position. The medical fluid thus can flow downstream through the connector 200.

When the pressure becomes equal to the predetermined threshold, the distal force exerted onto the pressure area 50 overcomes the proximal force of the resilient member 7 and thus causes the slider 5 to reach the closing position and the plug 4 to close the fluid outlet 24. The medical fluid is thereby prevented from flowing through the connector 200. When the pressure of the medical fluid exerted onto the pressure area 50 is equal to or higher than the predetermined threshold, the medical fluid is prevented from flowing through the connector 200.

The pressure area 50, the predetermined pressure threshold and the travel distance of the slider 5 between the opening position shown in Figure 2 and the closing position shown in Figure 6 may be determined according to the volume of medical fluid to be infused, and/or the dimensions of the medical container 100 (for example the inner diameter of the container). For example, the area of the pressure area 50 may be comprised between 30 mm² and 35 mm², preferably around 33 mm². The predetermined threshold may be around or equal to 25 psi, i.e. between 1,7.10⁵ Pa and 1,8.10⁵ Pa. The travel distance of the slider 5 between the opening position shown in Figure 2 and the closing position shown in Figure 6 may be for example around 2 mm.

The pressure area 50 may be defined by a proximal surface of a disk-shaped actuation member 52 of the slider 5. The pressure area 50 is preferably orthogonal to the longitudinal axis A. As shown on Figure 4 or 6, the slider 5, and more specifically the actuation member 52, is configured to let the medical fluid flow inside the internal conduit 20 without blocking said medical fluid, whatever the position of the slider 5.

The actuation member 52 may thus comprise at least one, and preferably two through-holes 54 allowing the medical fluid to pass through the pressure area 50. The through-holes 54 are preferably symmetric, with regard to a longitudinal plane including the central longitudinal axis A, so that the flow of the medical fluid remains symmetric. In an alternative embodiment not shown, a peripheral edge 56 of the actuation member 52 may include notches allowing the medical fluid to pass by the slider 5, and more precisely between the slider 5 and an inner surface of the internal conduit 20.

The resilient member 7 may be in the form of a bellow. In the absence of the medical fluid circulating inside the device 1, the resilient member 7 maintains the slider 5 and the plug 4 in the opening position as shown in Figure 2, which is the proximal-most position of the slider 5. The resilient member 7 is however designed to let the slider 5 and the plug 4 reach the closing position when the medical fluid pressure reaches or exceeds the predetermined threshold.

The slider 5 includes a connecting member, such as an axially extending connecting rod 58, in order to connect the pressure area 50 to the plug 4. The actuation member 52 may be integral with the connecting rod 58, such that the actuation member 52 and the connecting rod 58 form a single piece.

With reference to Figure 3, the plug 4 may however be a separate component that is secured to the connecting member for example by friction force or via a glue material. The plug 4 may comprise a hole 40 for receiving the connecting rod 58 and, if need be, said glue material.

In the illustrated embodiment, the actuation member 52 is provided at the proximal end of the slider 5, more specifically at the proximal end of the connecting rod 58. The plug 4 is secured to a distal end of the slider 5, and more specifically at the distal end of the connecting rod 58. The resilient member 7 may be arranged around the connecting rod 58. The connecting rod 58 thus extends through the resilient member 7.

With reference to Figures 2 and 3, the holder 8 comprises a guiding element configured to guide the translational movement of the slider 5 along the longitudinal axis A. The guiding element may be in the form of an inner tubular ring 80 shaped to receive the rod 58 of the slider 5 so that the rod 58 slides into the inner tubular ring 80.

It should be noted that a peripheral edge 56 of the slider 5, and more specifically a peripheral edge 56 of the actuation member 52 may be configured to contact an inner wall of the internal conduit 20 when the slider 5 moves inside the internal conduit 20, such that this peripheral edge 56 helps with guiding the slider 5 along the longitudinal axis A.

The resilient member 7 has one end supported by the inner tubular ring 80 of the holder 8 and an opposite end bearing against a distal face of the actuation member 52.

The holder 8 may comprise holding elements such as radial spokes 82 for connecting the inner tubular ring 80 to the housing 2 without hampering the flow of the medical fluid inside the internal conduit 20. The radial spokes 82 thus delimit through openings 86 for the circulation of fluid through the holder 8. The spokes 82 may be regularly distributed in a circumferential direction.

With reference to Figure 3, the housing 2 may comprise a top housing 30 and a bottom housing 32, and the holder 8 may comprise an outer ring 84 that may form part of the housing 2. The top housing 30 may be secured to a proximal face of this outer ring 84 while the bottom housing 32 may be secured to a distal face of the outer ring 84.

With reference to Figure 7, the proximal and distal connecting means are configured such that the connection between the device 1 and the medical container 100 and the connection between the device 1 and the connector 200 are removable. The device 1 is thus a separate component which is not integral with the medical container 100 or the connector 200. The healthcare practitioner is accordingly free to decide whether removing or not the device 1 as needed.

The proximal connecting means may comprise a female fitting 260, which may be in the form of a tubular portion, configured to tightly receive a male fitting corresponding to the cylindrical or conical distal tip 102 of the medical container 100. Furthermore, the proximal connecting means may comprise a connection feature, such as two diametrically opposed outer wings 262, designed to engage the inner thread 104 of an adaptor 106 mounted onto the distal tip 102 of the medical container 100. Therefore, the proximal connecting means are also able to cooperate with any standard syringe, such as 3 mL, 5mL or 10mL syringes, and may especially establish either a Luer slip or a Luer lock connection with this medical container 100.

As visible on Figures 1, 2 and 7, the distal connecting means may comprise a male fitting 280, which may be in the form of a cylindrical or conical tubular tip, configured to tightly engage a female fitting 202 of the connector 200. The distal connecting means may also comprise a collar 282 provided with an inner thread 284 for engaging a corresponding outer thread 204 arranged on the female fitting 202 of the connector 200. Accordingly, the distal connecting means are also preferably designed to cooperate with any standard fitting of the connector 200.

With reference for example to Figure 4, the internal conduit 20 may comprise a first section 20a having a first diameter, a second section 20b distal to the first section 20a and having a second diameter, and a third section 20c distal to the second section 20b and having a third diameter. The second diameter may be greater than the first diameter. The third diameter may be lower than the second diameter and possibly lower than the first diameter. The first and second sections 20a, 20b may be separated by a proximal tapered section 20d. In the same way, the second and third sections 20b, 20c may be separated by a distal tapered section 20e. In the opening position of Figure 2, the actuation member 52 may abut against the proximal tapered section 20d. In the closing position, the plug 4 may abut against the distal tapered section 20e. In an intermediate position (Figure 4), the pressure area 50 and the plug 4 may be remote from the tapered sections 20d, 20e.

In an embodiment, the plug 4 and the resilient member 7 are made of a softer material than the housing 2, the slider 5 and the holder 8. For example, the plug 4 and/or the resilient member 7 may be made of a rubber or a silicone material. The housing 2, the slider 5 and/or the holder 8 may be made of polypropylene or polycarbonate. In an embodiment, the plug 4, the resilient member 7, the slider 5, the holder 8 and the housing 2, 28 may be made by injection molding.

With reference to Figure 2, the plug 4 may include a distal tapered portion 42 to seal the fluid outlet 24.

The operation of the device 1 is described below with reference to Figures 2, 4, 5 and 6. In the absence of a medical fluid circulating inside the device 1 (Figure 2), the resilient member 7 maintains the slider 5 in a position corresponding to the proximal-most position of the slider 5. The plug 4 is remote from the fluid outlet 24.

When the user pushes the plunger rod 112 of the medical container 100 for infusing the medical fluid, a distal pressure is exerted on the pressure area 50 (arrows 51 on Figure 5). This distal pressure pushes the slider 5 in the distal direction, thereby compressing the resilient member 7. As long as the fluid pressure exerted on the pressure area 50 is lower than the predetermined threshold, the slider 5 is maintained by the resilient member 7 in an opening position (Figure 4) that is intermediate between the opening position of Figure 2 and the closing position of Figure 6. The plug 4 stays remote from the fluid outlet 24 so that the medical fluid is allowed to flow through the fluid outlet 24 towards the connector 200 (see arrows 240).

When the fluid pressure exerted on the pressure area 50 reaches or exceeds the predetermined threshold, the distal force exerted on the actuation member 52 overcomes the proximal force of the resilient member 7 such that the slider 5 is caused to move further in the distal direction until reaching the closing position, wherein the plug 4 closes the fluid outlet 24 (Figure 6). The medical fluid is thus blocked inside the device 1 and cannot flow in the connector 200. The healthcare practitioner infusing the medical product has thus to reduce the pressure exerted by his fingers on the plunger rod 112 in order to pursue the infusion.

When the fluid pressure exerted on the pressure area 50 decreases and becomes lower than the predetermined threshold again, the resilient member 7 pushes the slider 5 back towards the opening position (Figure 2 or 4). The plug 4 moves proximally away from the fluid outlet 24 and again allows the medical fluid to exit the device 1 and circulate in the connector 200.

With reference to Figure 7, the invention further relates to a medical container 100, such as prefilled or pre-fillable syringe, including a barrel 108 defining a reservoir containing a medical product, a distal tip 102 defining a passageway in fluid communication with said reservoir, and the above-described device 1 connected to said distal tip 102. The medical container 100 may comprise a plunger 110 and a plunger rod 112 for expelling the medical product through the distal tip 102. The medical container 100 preferably includes an adaptor 106 mounted onto said distal tip 102, said adaptor 106 including connecting means, such as an inner thread 104, engaging the proximal connecting means provided at the proximal end of the device 1 in order to secure the connection.

The device of the invention may also be connected to a connector 200, such as a needle hub or a vascular access device, for instance an intravenous (IV) catheter or more specifically a peripheral intravenous (PIV) catheter. The above-described device 1 is thus connected to a female fitting 202 of said connector 200. This female fitting 202 preferably has connecting means, such as an outer thread 204, engaging a threaded collar 282 of the device 1 in order to secure the connection. The device 1 may further be connected to the medical container 100.

The invention also relates to a method for manufacturing the above-described device 1, said method comprising the steps of:
(i) assembling the holder 8, the slider 5, the resilient member 7 and the plug 4;
(ii) securing the holder 8 to the bottom housing 32, preferably by ultrasonic welding;
(iii) securing the holder 8 to the top housing 30, preferably by ultrasonic welding.

In step (i), the resilient member 7 may be firstly positioned around the rod 58 of the slider 5. The rod 58 may then be inserted inside the inner tubular ring 80 of the holder 8. Then the plug 4 may be secured to the slider 5, for example by gluing.

The device 1 of the invention thus provides an easy mountable add-on component that allows controlling the infusion and/or injection pressure. The device 1 prevents occlusions in catheters and damage to blood vessels. The device 1 works with any syringe, catheter, needle or NFC (needle free connection) device, thereby making the device 1 universal. The healthcare practitioner can further remove the pressure control function by simply disconnecting the device 1.

## Claims

1. Device (1) for fluidly connecting a medical container (100) containing a medical fluid to a connector (200), wherein the device (1) includes
a housing (2), said housing (2) defining an internal conduit (20) for circulation of the medical fluid from a fluid inlet (22) to a fluid outlet (24),
a slider (5) arranged inside the internal conduit (20) such that the slider (5) is movable along a central longitudinal axis A between an opening position, wherein the medical fluid is allowed to flow through the fluid outlet (24), and a closing position, wherein the medical fluid is prevented from flowing through said fluid outlet (24), the slider (5) including a connecting rod (58) extending along the central longitudinal axis A, said connecting rod (58) connecting an actuation member (52) to a plug (4), said plug (4) being connected to the slider (5) and being configured to close the fluid outlet (24) when the slider (5) is in the closing position,
a resilient member (7) disposed proximally to the plug (4) and configured to exert a proximal force on said the slider (5) such that the resilient member (7) maintains the slider (5) in the opening position,
a proximally oriented pressure area (50) provided on the actuation member (52) of the slider (5), said pressure area (50) being configured to cause the slider (5) to reach the closing position against the action of the resilient member (7) when the medical fluid pressure exerted upon said pressure area (50) is equal to or higher than a predetermined threshold, thereby causing the plug (4) to close the fluid outlet (24), and
a holder (8), said holder (8) including a guiding element (80) for guiding the connecting rod (58) along the central longitudinal axis A, an outer ring (84) forming part of the housing (2), and holding elements (82) connecting the guiding element (80) to the outer ring (84) such that the holding elements (82) delimit openings (86) for allowing the medical fluid to flow through the holder (8).

2. Device according to the preceding claim, wherein the actuation member (52) includes a peripheral edge (56) that is configured to guide the sliding movement of the slider (5) between the opening and the closing positions, and the pressure area (50) delimits at least two through-holes (54) allowing the medical fluid to pass through the actuation member (52).

3. Device according to the preceding claim, wherein the at least two through-holes (54) are symmetric relative to a longitudinal plane containing the central longitudinal axis A.

4. Device according to any of the preceding claims, wherein the actuation member (52) is disk shaped.

5. Device according to any of the preceding claims, wherein the pressure area (50) is orthogonal to the central longitudinal axis A.

6. Device according to any of the preceding claims, wherein the actuation member (52) is integral with the connecting rod (58) such that the actuation member (52) and the connecting rod (58) form a single piece.

7. Device according to any of the preceding claims, wherein the resilient member (7) is a bellow extending around the connecting rod (58).

8. Device according to any of the preceding claims, wherein the resilient member (7) extends between the actuation member (52) and the guiding element (80) of the holder (8).

9. Device according to any of the preceding claims, wherein the housing (2) comprises a top housing (30) and a bottom housing (32), the outer ring (84) of the holder (8) forming an intermediate housing (2) portion interposed between the top and the bottom housings (30, 32).

10. Device according to any of the preceding claims, wherein the plug (4) is made of a softer material than the slider (5).

11. Combination comprising the device (1) according to any of the preceding claims and the medical container (100) comprising a reservoir for containing a medical fluid, the device (1) being fluidly connected to the reservoir of said medical container (100).

12. Method for manufacturing the device (1) according to claim 9, said method comprising the steps of:
assembling the holder (8), slider (5), the resilient member (7) and the plug (4);
securing the holder (8) to the bottom housing (32), preferably by ultrasonic welding;
securing the holder (8) to the top housing (30), preferably by ultrasonic welding.

## Patentansprüche

1. Vorrichtung (1) zum fluidischen Verbinden eines medizinischen Behälters (100), der ein medizinisches Fluid enthält, mit einem Verbindungsstück (200), wobei die Vorrichtung (1) enthält
ein Gehäuse (2), wobei das Gehäuse (2) einen inneren Kanal (20) für die Zirkulation des medizinischen Fluids von einem Fluideinlass (22) zu einem Fluidauslass (24) definiert,
einen Schieber (5), der innerhalb des inneren Kanals (20) so angeordnet ist, dass der Schieber (5) entlang einer zentralen Längsachse A zwischen einer Öffnungsposition, in der das medizinische Fluid durch den Fluidauslass (24) fließen kann, und einer Schließposition, in der das medizinische Fluid daran gehindert wird, durch den Fluidauslass (24) zu fließen, beweglich ist, wobei der Schieber (5) eine Verbindungsstange (58) enthält, die sich entlang der zentralen Längsachse A erstreckt, wobei die Verbindungsstange (58) ein Betätigungselement (52) mit einem Stopfen (4) verbindet,
wobei der Stopfen (4) mit dem Schieber (5) verbunden und so eingerichtet ist, dass er den Fluidauslass (24) verschließt, wenn sich der Schieber (5) in der Schließposition befindet,
ein elastisches Element (7), das proximal zum Stopfen (4) angeordnet und so eingerichtet ist, dass es eine proximale Kraft auf den Schieber (5) ausübt, so dass das elastische Element (7) den Schieber (5) in der Öffnungsposition hält,
einen proximal ausgerichteten Druckbereich (50), der an dem Betätigungselement (52) des Schiebers (5) vorgesehen ist, wobei der Druckbereich (50) so eingerichtet ist, dass er bewirkt, dass der Schieber (5) gegen die Wirkung des elastischen Elements (7) die Schließposition erreicht, wenn der auf den Druckbereich (50) ausgeübte medizinische Fluiddruck gleich oder höher als ein vorbestimmter Schwellenwert ist, wodurch der Stopfen (4) den Fluidauslass (24) verschließt, und
einen Halter (8), wobei der Halter (8) ein Führungselement (80) zum Führen der Verbindungsstange (58) entlang der zentralen Längsachse A, einen Außenring (84), der ein Teil des Gehäuses (2) ist, und Halteelemente (82) enthält, die das Führungselement (80) mit dem Außenring (84) verbinden, so dass die Halteelemente (82) Öffnungen (86) begrenzen, um zu ermöglichen, dass das medizinische Fluid durch den Halter (8) fließt.

2. Vorrichtung nach dem vorhergehenden Anspruch, wobei das Betätigungselement (52) eine Umfangskante (56) enthält, die so eingerichtet ist, dass sie die Schiebebewegung des Schiebers (5) zwischen der Öffnungs- und der Schließposition führt, und der Druckbereich (50) mindestens zwei Durchgangslöcher (54) begrenzt, die es ermöglichen, dass das medizinische Fluid durch das Betätigungselement (52) strömt.

3. Vorrichtung nach dem vorhergehenden Anspruch, wobei die mindestens zwei Durchgangslöcher (54) relativ zu einer Längsebene, die die zentrale Längsachse A enthält, symmetrisch sind.

4. Vorrichtung nach einem der vorhergehenden Ansprüche, wobei das Betätigungselement (52) scheibenförmig ist.

5. Vorrichtung nach einem der vorhergehenden Ansprüche, wobei der Druckbereich (50) orthogonal zu der zentralen Längsachse A ist.

6. Vorrichtung nach einem der vorhergehenden Ansprüche, wobei das Betätigungselement (52) fest mit der Verbindungsstange (58) verbunden ist, so dass das Betätigungselement (52) und die Verbindungsstange (58) ein Stück bilden.

7. Vorrichtung nach einem der vorhergehenden Ansprüche, wobei das elastische Element (7) ein sich um die Verbindungsstange (58) erstreckender Faltenbalg ist.

8. Vorrichtung nach einem der vorhergehenden Ansprüche, wobei sich das elastische Element (7) zwischen dem Betätigungselement (52) und dem Führungselement (80) des Halters (8) erstreckt.

9. Vorrichtung nach einem der vorhergehenden Ansprüche, wobei das Gehäuse (2) ein oberes Gehäuse (30) und ein unteres Gehäuse (32) umfasst, wobei der Außenring (84) des Halters (8) einen Zwischenabschnitt des Gehäuses (2) bildet, der zwischen dem oberen und dem unteren Gehäuse (30, 32) eingefügt ist.

10. Vorrichtung nach einem der vorhergehenden Ansprüche, wobei der Stopfen (4) aus einem weicheren Material als der Schieber (5) besteht.

11. Kombination, umfassend die Vorrichtung (1) nach einem der vorhergehenden Ansprüche und den medizinischen Behälter (100), umfassend ein Reservoir zur Aufnahme eines medizinischen Fluids, wobei die Vorrichtung (1) fluidisch mit dem Reservoir des medizinischen Behälters (100) verbunden ist.

12. Verfahren zur Herstellung der Vorrichtung (1) nach Anspruch 9, wobei das Verfahren die folgenden Schritte umfasst:
Zusammenbauen des Halters (8), des Schiebers (5), des elastischen Elements (7) und des Stopfens (4);
Befestigen des Halters (8) an dem unteren Gehäuse (32), vorzugsweise durch Ultraschallschweißen;
Befestigen des Halters (8) an dem oberen Gehäuse (30), vorzugsweise durch Ultraschallschweißen.

## Revendications

1. Dispositif (1) pour relier de manière fluidique un récipient médical (100) contenant un fluide médical à un raccord (200), dans lequel le dispositif (1) comprend
un boîtier (2), ledit boîtier (2) définissant un conduit interne (20) pour la circulation du fluide médical d'une entrée de fluide (22) à une sortie de fluide (24),
un coulisseau (5) agencé à l'intérieur du conduit interne (20) de sorte que le coulisseau (5) puisse se déplacer le long d'un axe longitudinal central A entre une position d'ouverture, dans laquelle le fluide médical est autorisé à s'écouler à travers la sortie de fluide (24), et une position de fermeture, dans laquelle le fluide médical est empêché de s'écouler à travers ladite sortie de fluide (24), le coulisseau (5) comprenant une tige de liaison (58) s'étendant le long de l'axe longitudinal central A, ladite tige de liaison (58) reliant un élément d'actionnement (52) à un bouchon (4),
ledit bouchon (4) étant relié au coulisseau (5) et étant configuré pour fermer la sortie de fluide (24) lorsque le coulisseau (5) est dans la position de fermeture,
un élément élastique (7) disposé à proximité du bouchon (4) et configuré pour exercer une force proximale sur ledit coulisseau (5) de sorte que l'élément élastique (7) maintienne le coulisseau (5) dans la position d'ouverture,
une zone de pression orientée de manière proximale (50) prévue sur l'élément d'actionnement (52) du coulisseau (5), ladite zone de pression (50) étant configurée pour amener le coulisseau (5) à atteindre la position de fermeture contre l'action de l'élément élastique (7) lorsque la pression de fluide médical exercée sur ladite zone de pression (50) est supérieure ou égale à un seuil prédéterminé, amenant ainsi le bouchon (4) à fermer la sortie de fluide (24), et
un support (8), ledit support (8) comprenant un élément de guidage (80) pour guider la tige de liaison (58) le long de l'axe longitudinal central A, une bague externe (84) formant partie du boîtier (2) et des éléments de maintien (82) reliant l'élément de guidage (80) à la bague externe (84) de sorte que les éléments de maintien (82) délimitent des ouvertures (86) pour permettre au fluide médical de s'écouler à travers le support (8).

2. Dispositif selon la revendication précédente, dans lequel l'élément d'actionnement (52) comprend un bord périphérique (56) qui est configuré pour guider le mouvement de coulissement du coulisseau (5) entre les positions d'ouverture et de fermeture, et la zone de pression (50) délimite au moins deux trous traversants (54) permettant au fluide médical de passer à travers l'élément d'actionnement (52).

3. Dispositif selon la revendication précédente, dans lequel les au moins deux trous traversants (54) sont symétriques par rapport à un plan longitudinal contenant l'axe longitudinal central A.

4. Dispositif selon l'une des revendications précédentes, dans lequel l'élément d'actionnement (52) est en forme de disque.

5. Dispositif selon l'une des revendications précédentes, dans lequel la zone de pression (50) est orthogonale à l'axe longitudinal central A.

6. Dispositif selon l'une des revendications précédentes, dans lequel l'élément d'actionnement (52) est solidaire de la tige de liaison (58) de sorte que l'élément d'actionnement (52) et la tige de liaison (58) forment une seule pièce.

7. Dispositif selon l'une des revendications précédentes, dans lequel l'élément élastique (7) est un soufflet s'étendant autour de la tige de liaison (58).

8. Dispositif selon l'une des revendications précédentes, dans lequel l'élément élastique (7) s'étend entre l'élément d'actionnement (52) et l'élément de guidage (80) du support (8).

9. Dispositif selon l'une des revendications précédentes, dans lequel le boîtier (2) comprend un boîtier supérieur (30) et un boîtier inférieur (32), la bague externe (84) du support (8) formant une partie de boîtier intermédiaire (2) interposée entre les boîtiers (30, 32) supérieur et inférieur.

10. Dispositif selon l'une des revendications précédentes, dans lequel le bouchon (4) est réalisé en un matériau plus souple que le coulisseau (5).

11. Combinaison comprenant le dispositif (1) selon l'une des revendications précédentes et le récipient médical (100) comprenant un réservoir pour contenir un fluide médical, le dispositif (1) étant relié de manière fluidique au réservoir dudit récipient médical (100).

12. Procédé de fabrication du dispositif (1) selon la revendication 9, ledit procédé comprenant les étapes :
d'assemblage du support (8), du coulisseau (5), de l'élément élastique (7) et du bouchon (4) ;
de fixation du support (8) au boîtier inférieur (32), de préférence par soudage par ultrasons ;
de fixation du support (8) au boîtier supérieur (30), de préférence par soudage par ultrasons.
